# EUROPEAN PATENT APPLICATION

(11) **EP 2 127 615 A2**
(43) Date of publication of application: **02.12.2009**
(21) Application number: 09170456.9
(22) Date of filing: 16.01.2007
(51) Int. Cl.: A61F 2/06

(54) **System of Attaching Vessels to Grafts**

(30) Priority: 01.02.2006 US 344682
(62) Divisional of application: 07250163.8
(71) Applicant: Cordis Corporation, Miami Lakes, FL 33014 (US)
(72) Inventor: Duerig, Thomas W., Fremont, CA 94539 (US)
(74) Representative: Brown, George Laurence

(57) **Abstract**

A suture-less graft coupling system for anastamosis and bypass procedures comprising a first stent for insertion into a radially transected vessel being treated, a graft member overlapping the stented area of the transected vessel, a second stent overlapping the graft and vessel in an area of the first stent wherein sealing pressure from the second stent can be applied to seal the vessel and graft in a leak-free arrangement, and a third stent wherein the graft receives the third stent and is inserted into the transacted vessel.

## Description

This invention relates generally to medical devices for attaching vessels to grafts. More particularly, the invention relates to non-suture devices for attaching grafts to vessels in by-pass operations and the like using cooperating sealing stents.

Endovascular grafts, stents and like devices are known in the art for aiding, repairing or bypassing blood flow through vascular vessels. As will be appreciated, these prior art devices require skill and precision during surgery to deliver and properly couple the device to the vasculature of the patient. Take bypass grafts for example. Aside from the difficulties associated with delivering the graft to the proper location, heretofore, in typical bypasses, for example, the bypass graft must be manually sutured to the native vessel. Surgically suturing the graft is a tedious and time consuming process, requiring substantial skill and experience to achieve a secure and leak-free coupling. Undesirable results, such as leaks, are not uncommon, and require the suturing to be modified or supplemented. The adverse consequences posed by suturing are clear drawbacks with these prior art grafts and like devices.

These drawbacks are particularly acute in femoral-femoral (Fem-Fem) or femoral-popilteal(Fem-Pop) bypasses. While many vascular procedures, such as Fem-Fem and Fem-Pop by-pass operations involve suturing an artificial or harvested graft onto a blood vessel, typically, in Fem-Fem or Fem-Pop bypasses, this is done by longitudinally slitting the vessel (after clamping upstream from the slit), shaping the end of the graft to fit the opening in the vessel, and then suturing around the circumference of the graft end and into the native vessel. This process of suturing two vessels together, known in the art as anastamosis, requires a great deal of time and attention to assure that the suturing is free of leaks and sufficiently strong given the length and orientation of the longitudinal slit. As will be appreciated, one drawback of this procedure is that it takes even experienced surgeons a lot of time, up to 15 minutes per anastamosis, to perform given the length and orientation of the longitudinal slit. Moreover, another drawback is that even skilled surgeons taking their time are often unable to produce consistently leak-free results over the entire longitudinal slit given the number of sutures involved.

Accordingly, there exists a long-felt, yet unresolved need in the art for improved devices and methods for endovascular treatment and repairs, such as coupling grafts and bypass grafts and the like to native vessels, without the need for suturing. Moreover, there exists a need in the art for a method of performing bypass surgeries, such as Fem-Fem or Fem-Pop bypasses, without longitudinal slitting the vessel.

The present invention overcomes the practical problems described above and offers new advantages as well. One object of the invention is to provide a sutureless method of joining two vessels. Another object of the invention is to provide a sutureless method of coupling an artificial graft to a native vessel. Another obj ect of the invention is to provide devices and methods for coupling vasculature end-to-end. Yet another object of the invention is to provide a sutureless method of performing Fem-Fem and Fem-Pop bypass operations. It is still a further object of this invention of providing a method of performing Fem-Fem and Fem-Pop or like bypass operations without the need to longitudinal slit the vessel.

These and other objects and advantages of the present invention may be realized by a graft coupling device and system comprising a first intra-luminal implant for insertion in a vessel to be treated, a graft member for accepting at least a portion of the implanted-vessel, and a second intra-luminal implant configured for positioning around at least a portion of the implant-graft intersection, wherein said second intra-luminal implant is adapted to seal said vessel and graft in a leak-free hold.

These and other objects and advantages of the present invention may be realized by a method of coupling a graft to a vessel comprising the steps of clamping the vessel upstream from the target anastamosis site, circumferentially transecting the vessel at the anastamosis site, inserting a first intra-luminal implant into the transected vessel, wherein the implant acts as a support structure for the vessel and preferably has radius approximates the radius of the vessel after deployment, sliding the graft vessel over the transected-end of the transected and supported vessel, and placing a second intra-luminal implant around the area of the overlap of the vessel and the graft, wherein said second implant is configured to provide a sealing force to the area.

An advantageous feature of at least one embodiment of the invention is that the first intra-luminal implant serves as a supporting framework for the vessel or graft in which it is inserted. Preferable intra-luminal implants include those that are configured as a collapsible and expandable supporting framework, such as a matrix of struts or a stent. A preferred stent for use with the present invention is an expandable stent, such as a stent made from a shape-memory alloy such as nitinol or the like, that is sized to approximate the size of the vessel being repaired. This sizing may allow a graft vessel to more easily fit over the stented section of the transected vessel. According to this feature of the invention, the first intra-luminal device, or support structure, may be balloon expandable such as many common stents, or alternately, simply fit into place.

Another advantageous feature of at least one embodiment of the invention is that the first intra-luminal implant may be deployed into the vessel prior to radial transection so as to provide more support for a precise transection. Such deployment could be accomplished via a direct-stick method, such as would be used in the case of an expandable stent.

Another advantageous feature of at least one embodiment of the invention is that the second intra-luminal implant may be shrinkable or radially restricted from a first diameter to a second diameter to apply pressure to the supported-vessel and graft intersection. Such restriction may be due to elastic springback, or alternately, the result of shape-memory shrinking initiated via warming by the body's natural temperature. Preferably, according to the invention, the forces of the two intra-luminal implants can be carefully controlled so as to provide a high integrity seal without reaching pressures that may cause necrosis.

Another advantageous aspect of at least one embodiment of the invention is that is possible to reverse the position of the vessel and graft, wherein the graft is inserted inside the supported vessel. Another advantageous aspect of at least one embodiment of the invention is that is possible to complete and seal the anastamosis when the two implants are not overlapped or when the first implant extends beyond the vessel transaction point. According to yet another advantageous aspect of at least one embodiment of the invention, one or both intra-luminal implants may incorporate barbs or other anchoring means to prevent axial movement of the graft and vessel.

In one embodiment, the first intra-luminal implant and said second intra-luminal implant cooperate to provide sealing pressure less than that which may cause necrosis.

Embodiments of the invention will now be described by way of example with reference to the accompanying drawings, where like reference numbers indicate identical or functionally similar elements, and in which:
FIG. 1 is an illustration of a clamped and transected vessel about to receive a stent according to the invention.
FIG. 2 is an illustration of a stented vessel configured to be inserted in a graft vessel according to the invention.
FIG. 3 is an illustration of an outer stent positioned over an area of overlap of a stented vessel and graft vessel intersection according to the invention.
FIG. 4 is a cross-sectional view of a sealed stented vessel and graft anastamosis according to the invention.

The present invention is based, in part, on the discovery that internal and external intra-luminal implants, such as stent-like structures, may be adapted to serve as supports and seals to facilitate sutureless coupling of two or more vessels. While the present invention will be described in connection with end-to-end anastamosis methods and devices for achieving Fem-Fem and Fem-Pop bypasses, one of ordinary skill in the art will readily appreciate that the present invention may be adapted for numerous uses in a variety of fields. For example, the present invention may be useful in any bypass procedure and may be useful in any environment where end to end or radially transected vessel are treated. Moreover, while the present invention will be described using expandable and retractable stents, the invention should be understood to incorporate any intra-luminal implant that is capable of serving as a support structure for a vessel into which it is to be inserted and any intra-luminal implant that is capable of providing a seal in the area of interest.

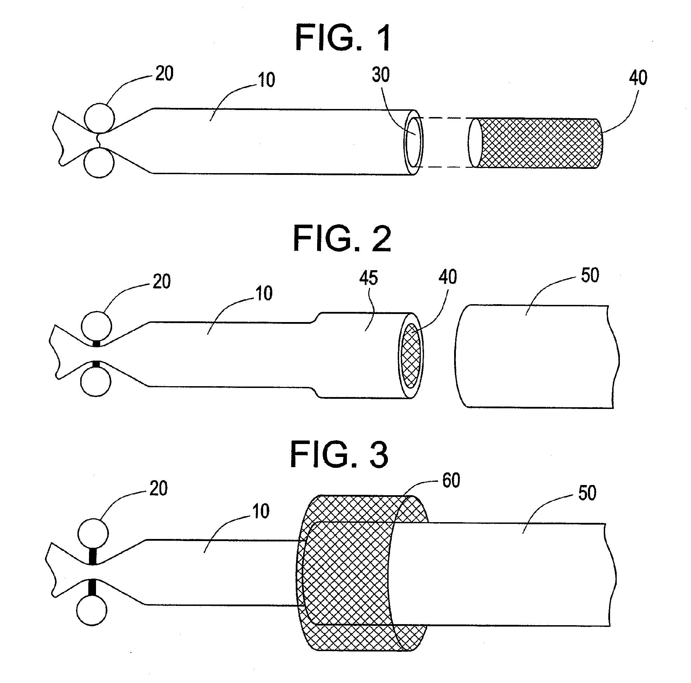

Figures 1-4 depict a preferred embodiment and method of joining a transected vessel and a bypass graft. As shown in Fig. 1, a vessel 10 to be treated is clamped in a region upstream of the area to be bypassed via a compression clamp 20. The vessel is radially transected to define a radial vessel opening 30. A first stent member 40 is sized and positioned in said radial opening 30. A preferred embodiment of the first stent 40 is a nitinol stent having a diameter approximating that of the inside vessel diameter. However, any suitable stent comprised of any suitable material may be used according to the invention. The ability to provide a rigid conduit for blood flow is the primary consideration.

If the first stent 40 is inserted into the vessel opening 30 after the radial transection of the vessel, then the stent 40 may simply be configured to fit into place. However, in an alternate embodiment, it may be preferable for the first stent to be deployed into the vessel prior to the radial transection so as to provide additional support for a precise transection. According to this embodiment of the invention, the stent may be a collapsible, catheter deployed stent that is balloon expandable to a desired diameter. The deployment could be done via a direct-stick method or any other suitable delivery and deployment method. Any suitable materials for construction of a suitable stent may be used, including shape-memory materials, such as nitinol, or any other collapsible and expandable strut or framework configuration such as those commonly used in the art.

As illustrated in Fig. 2, after the first stent 40 is positioned inside the vesse's radial opening 30, the graft vessel 50 is advanced over the stented area 45. Any suitable method of advancing the graft 50 over the stented area 45 may be used according to the invention. Likewise, the exact positioning of the graft 50 is not critical to the invention. Unlike prior art suturing methods of anastamoses procedures, the present invention allows some degree of flexibility in the procedure. For example, the graft may be advanced to an area beyond the stented area or an area only partly down the stented area. In other words, there does not need to be a precise overlap of the vessels.

Turning to Fig. 3, once the graft 50 is advanced over the stented area 45 to a suitable end-point, a second stent, functionally described as a "sealing stent" 60, is positioned over the area of the first stent 40. As depicted, the interior diameter of the sealing stent 60 surrounds at least a portion of the stented area 45 and at least a portion of its overlap with the graft 50. This configuration allows the radial retraction of the sealing stent 60 to apply a sealing pressure to a portion of the area where the vessels overlap or intersect. The cooperation of the outwardly biased inner stent 40 with the innerly biased sealing stent 60 operates to form a leak-free seal.

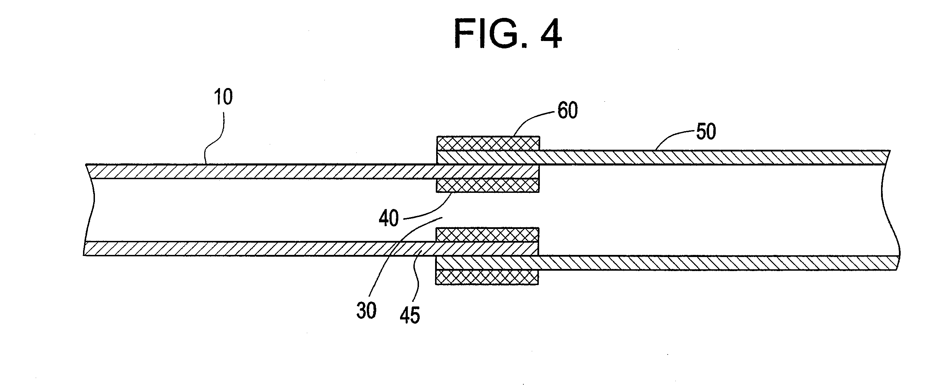

This cooperation is best shown in the cross-sectional view of Fig. 4. The sealing stent 60 may be any suitable stent or coupling that is configured to provide sealing pressure to the graft 50 in an area that overlaps the stented area 45. Again, it is not critical that the sealing stent 60 completely overlap the entire stented area. Any positioning that allows for stents 40, 60 to compress both the vessel 30 and graft 50 between the two stents may be used according to the invention.

In a preferred embodiment, the sealing stent 60 comprises a thermally shrinking stent, whereby the warming of the stent via the body's natural temperature causes the stent to provide inwardly directed pressure. Preferably, the stent has a generally O-shaped configuration whereby retraction via warming results in an even pressure applied around the circumference of the interior to any tubular object disposed therein. Alternatively, the sealing stent 60 could be manufactured of any suitable materials that result in an elastic springback from a larger first diameter (the stretched state) for positioning over the area of interest to a smaller second diameter (the compressing state) that applies interior pressure to the vessel and graft disposed therein.

In order to aid in the assurance of proper positioning and sealing of the anastamosis, it may be desirable to provide either or both stents with barbs in order to prevent axial movement of the graft and/or vessel during surgery. The use of barbs may also serve to anchor the composite in place after surgery to assure after the longevity of the sealing engagement of the vessels.

Also, while the cooperating stent configuration is preferably suited for providing suture-free anastomoses, there may be situations where sutures or the like are used in conjunction with the sealing arrangements described herein.

The applicability of the cooperating stents described above to other vascular operations is clear. Moreover, the use of the sutureless methods disclosed herein lends to crafting suitable alternate configurations of the components for an intended use. For example, in an alternative embodiment to that depicted in Figs. 1-4, it may be preferable to reverse the radial position of the vessel and graft, wherein the graft winds up being inside the native vessel. In that configuration, the graft may be stented or otherwise made rigid enough to resist collapsing from the sealing pressure of the outer stent. Likewise, it may also be preferably to use three stents, one for the vessel, one for the graft and one to seal and sandwich the composite. Also, there may be times when the two stents are not overlapped, or when the first stent extends beyond the transection point and the graft is advanced over the exposed area of the stent.

## Claims

1. A graft coupling system comprising:
a first stent for insertion in a transected vessel receiving a graft, said stent providing a rigid conduit for blood flow through said vessel and sized to allow a graft to be advanced to overlap at least part of a stented-area of said vessel;
a second stent for surrounding at least a part of said area of overlap between said graft and said stented area, said second stent configured to apply sealing pressure to said graft in a part of said area of overlap, whereby said first and second stents sandwich said graft to provide a fluid tight seal between said vessel and said graft; and
a third stent, wherein said graft receives said third stent and is inserted into said transected vessel.

2. The system of claim 1, wherein said first stent is sized to approximate the diameter of the transected vessel.

3. The system of claim 2, wherein said first stent comprises an expandable stent.

4. The system of claim 3, wherein said first stent comprises nitinol.

5. The system of claim 1, wherein said second stent applies sealing pressure via elastic springback.

6. The system of claim 1, wherein said second stent applies sealing pressure via thermal shrinking.

7. The system of claim 6, wherein said thermal shrinking is caused by warming said second stent to body temperature.

8. The system of claim 1, wherein said first stent includes barbs for anchoring said stent in said transected vessel.

9. The system of claim 1, wherein said second stent includes barbs for anchoring said stent to at least a portion of said graft.

10. The system of claim 1, wherein said first stent extends outside said vessel and into an interior of said graft.

11. The system of claim 1, wherein said first stent is configured to be deployed into said vessel prior to transection.
